# EUROPEAN PATENT APPLICATION

(11) **EP 1 836 965 A1**
(43) Date of publication of application: **26.09.2007**
(21) Application number: 06711539.4
(22) Date of filing: 11.01.2006
(51) Int. Cl.: A61B 8/12

(54) **ULTRASONIC ENDOSCOPE INSPECTION DEVICE**

(30) Priority: 11.01.2005 JP 2005004576
(71) Applicant: Olympus Corporation, Tokyo 151-0072 (JP)
(72) Inventor: YOSHIDA, Taizo c/o Olympus Medical Systems, Tokyo 1510072 (JP)
(74) Representative: von Hellfeld, Axel
(86) International application number: PCT/JP2006/300190
(87) International publication number: WO 2006/075603

(57) **Abstract**

The present invention automatically performs inspections including: inspection by a power source inspecting section of a power source that supplies power from a main ultrasonic observation unit to an ultrasonic endoscope; inspection by a transmission signal inspecting section of a transmission signal Tx and a 3D drive clock from the main ultrasonic observation unit to the ultrasonic endoscope; inspection by an encoder inspecting section of a Phase A and Phase Z; inspection by a code inspecting section of a scanner code and a frequency code from the ultrasonic endoscope 3 to the main ultrasonic observation unit; and inspection by a position sensor signal inspecting section of a position sensor signal from the ultrasonic endoscope to the main ultrasonic observation unit. In this way, failure conditions of the ultrasonic endoscope apparatus can be readily determined.

## Description

### Technical Field

The present invention relates to an inspection device for an ultrasonic endoscope apparatus, which inspects a main body and an ultrasonic endoscope of an ultrasonic endoscope apparatus to find failure therein.

### Background Art

An ultrasonic endoscope apparatus has been developed recently, which can observe the inside of a body cavity, or which, specifically, can observe the tissues in a living organism of the body cavity, through ultrasonic images by using an ultrasonic endoscope whose elongated insertion portion is provided with an ultrasonic transmitter/receiver element at its distal end.

In such an ultrasonic endoscope apparatus, spiral scanning is performed with the forward and rearward movement of the ultrasonic transmitter/receiver element provided at the distal end of the insertion portion of the ultrasonic endoscope to transmit/receive ultrasonic waves. Meanwhile, images are processed in a main ultrasonic observation unit of the apparatus, so that ultrasonic images of the tissues in the living body can be observed.

### Disclosure of Invention

### Means for Solving the Problem

Such an ultrasonic endoscope apparatus, however, has a failure mode peculiar to the apparatus, which necessitates carrying out the inspection of the apparatus, dissociating the possible failure that would be caused by the main ultrasonic observation unit from the possible failure that would be caused by the ultrasonic endoscope. For example, when an ultrasonic endoscope apparatus installed in a user facility, such as a hospital, is in failure, a determination has to be made at the user facility as to whether the main ultrasonic observation unit is in failure or whether the ultrasonic endoscope is in failure.

In a conventional method of failure diagnosis, a normal main ultrasonic observation unit and a normal ultrasonic endoscope have been brought to the user facility, where the ultrasonic endoscope that would be in failure is connected to the normal main ultrasonic observation unit, and the normal ultrasonic endoscope is connected to the main ultrasonic observation unit that would be in failure to determine whether the main ultrasonic observation unit is in failure or whether the ultrasonic endoscope is in failure. Thus, it has been required to bring a normal main ultrasonic observation unit and a normal ultrasonic endoscope into the user facility.

Another method of failure diagnosis that can be considered is to inspect every signal in an ultrasonic endoscope apparatus in failure by using a signal tester, such as an oscilloscope. However, carrying out this method at the user facility has not always enabled location of a failure. As a matter of fact, it has been necessary to bring both the ultrasonic endoscope and the main ultrasonic observation unit that would be in failure to the factory (or the service center), for example.

The present invention has been made in light of the circumstances described above, and has as its object to provide an inspection device for an ultrasonic endoscope apparatus, which is capable of readily determine the failure conditions of the ultrasonic endoscope apparatus.

### Brief Description of the Drawings

Fig. 1 is a block diagram illustrating a connection state of an inspection device for an ultrasonic endoscope apparatus according to a first embodiment of the present invention;
Fig. 2 is a block diagram illustrating an inspection circuit in the inspection device illustrated in Fig. 1;
Fig. 3 is an illustration of a configuration of an LED display panel in the inspection device illustrated in Fig. 1; and
Fig. 4 is a block diagram illustrating an inspection circuit in an inspection device according to a second embodiment of the present invention.

### Best Mode for Carrying Out the Invention

Hereinafter are described embodiments of the present invention with reference to the drawings.

### (First Embodiment)

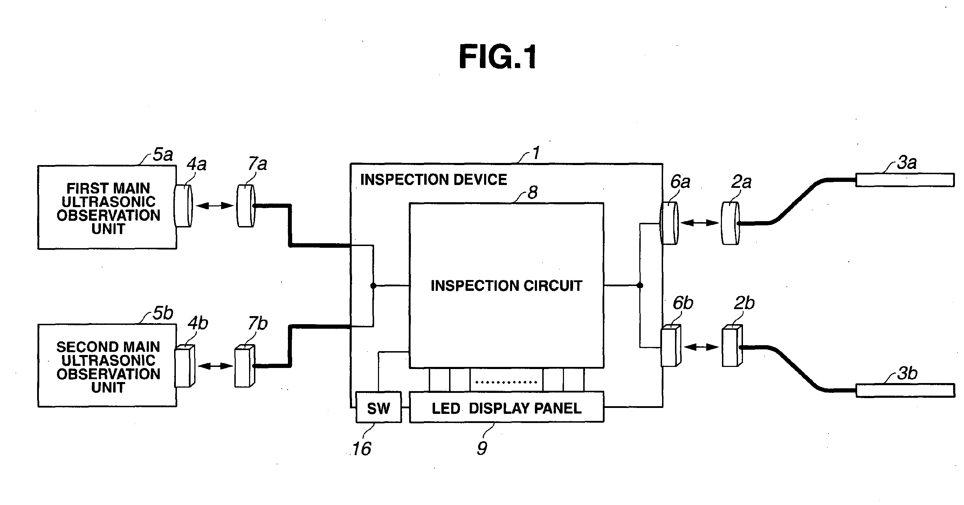

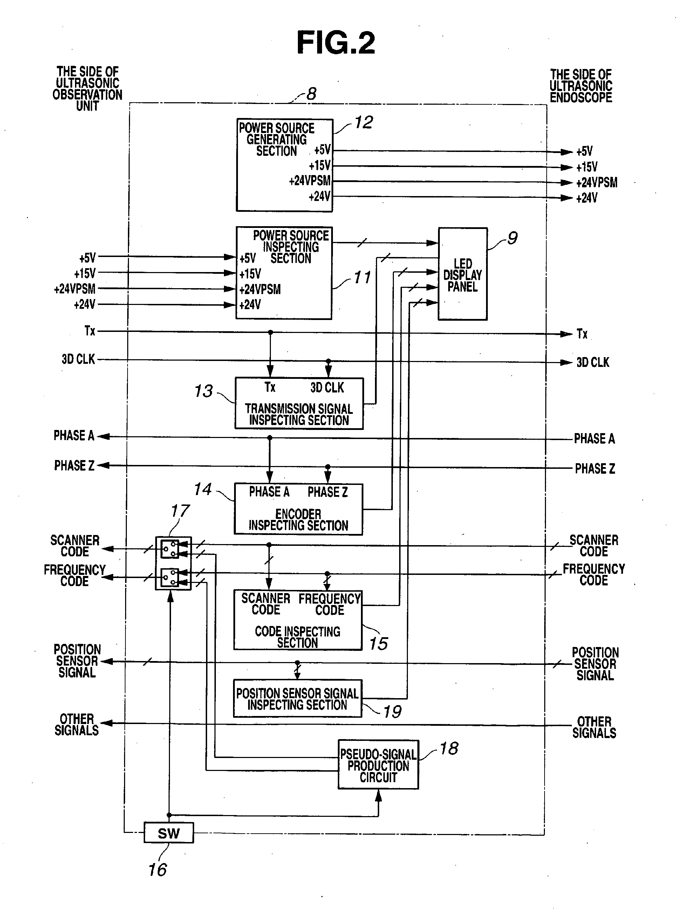

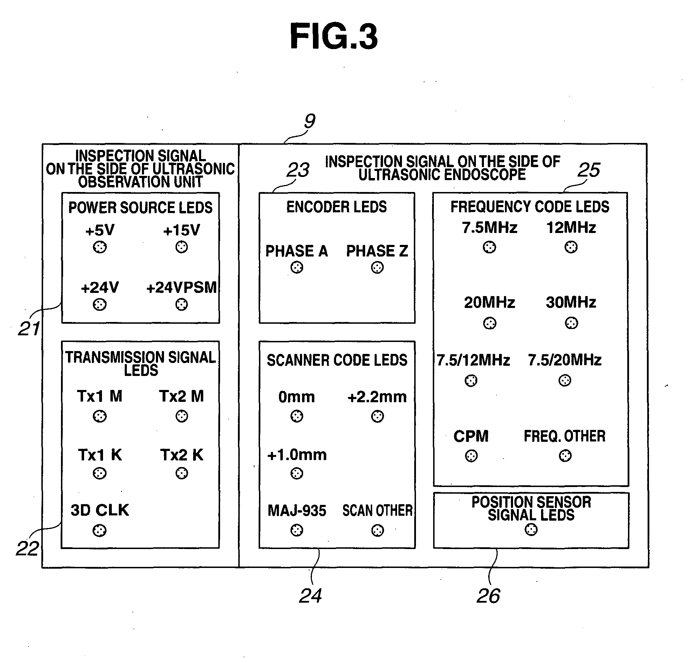

Figs. 1 to 3 relate to a first embodiment of the present invention. Fig. 1 is a block diagram illustrating a connection state of an inspection device for an ultrasonic endoscope apparatus. Fig. 2 is a block diagram illustrating an inspection circuit in the inspection device illustrated in Fig. 1. Fig. 3 is an illustration of a configuration of an LED display panel in the inspection device illustrated in Fig. 1.

As shown in Fig. 1, an inspection device 1 for an ultrasonic endoscope apparatus according to the present embodiment is configured such that an ultrasonic endoscope and a main ultrasonic observation unit having connectors of different shapes can be connected.

Specifically, the inspection device 1 of the ultrasonic endoscope apparatus has a round connector receiver 6a and a round connector 7a, to which, for example, a first ultrasonic endoscope 3 a having a round connector 2a at its proximal end and a first main ultrasonic observation unit 5a having a round connector receiver 4a to be connected to the first ultrasonic endoscope 3a, can be connected, respectively. Also, the inspection device 1 has a square connector receiver 6b and a square connector 7b, to which, for example, a second ultrasonic endoscope 3b having a square connector 2b at its proximal end and a second main ultrasonic observation unit 5b having a square connector receiver 4b to be connected to the second ultrasonic endoscope 3b, can be connected, respectively.

Further, the inspection device 1 of the ultrasonic endoscope apparatus includes: an inspection circuit 8 therein for inspecting conditions of signals, and the like, in the connected ultrasonic endoscopes and the main ultrasonic observation units; and an LED display panel 9 over an entire surface of the apparatus, which has a plurality of LEDs, for example, for displaying the results of the inspection carried out by the inspection circuit 8.

As shown in Fig. 2, the inspection circuit 8 includes: a power source inspecting section 11, which inspects a power source that supplies power from the first main ultrasonic observation unit 5a or the second main ultrasonic observation unit 5b (hereinafter referred to as "main ultrasonic observation unit 5") to the first ultrasonic endoscope 3a or the second ultrasonic endoscope 3b (hereinafter referred to as "ultrasonic endoscope 3"); and a power source generating section 12 for supplying electric power to the ultrasonic endoscope 3. The results of the inspection carried out by the power source inspecting section 11 are outputted to the LED display panel 9.

The inspection circuit 8 also includes: a transmission signal inspecting section 13 for inspecting a transmission signal Tx that defines oscillation timing of an ultrasonic pulse transmitted from the main ultrasonic observation unit 5 to the ultrasonic endoscope 3, in an ultrasonic transmitter/receiver element (not shown) which is incorporated in a distal end of the ultrasonic endoscope 3 and for inspecting a 3D drive clock (3D CLK) for spirally driving the ultrasonic transmitter/receiver element; and an encoder inspecting section 14 for inspecting a Phase A and a Phase Z, that is, an encoder signal (ultrasonic echo signal) outputted to the main ultrasonic observation unit 5 from the ultrasonic endoscope 3. The presence and the output level of the individual signals are inspected, the results of which are outputted to the LED display panel 9.

It should be appreciated that the transmission signal Tx and the 3D drive clock (3D CLK) are through-outputted from the main ultrasonic observation unit 5 to the ultrasonic endoscope 3, and the Phase A and the Phase Z are through-outputted from the ultrasonic endoscope 3 to the main ultrasonic observation unit 5.

The inspection circuit 8 also includes a code inspecting section 15 for inspecting a scanner code and a frequency code outputted from the ultrasonic endoscope 3 to the main ultrasonic observation unit 5. Specifically, an arrangement is so made that the scanning mode of the ultrasonic scanning performed by the ultrasonic endoscope 3 and the frequency of the ultrasonic waves produced by the ultrasonic endoscope 3 are inspected, and the results of the inspection are outputted to the LED display panel 9.

It should be appreciated that an output-selectable configuration is provided. Particularly, in the configuration, it is either that a scanner code and a frequency code are through-outputted from the ultrasonic endoscope 3 to the main ultrasonic observation unit 5 by controlling a switching section 17 with a switch 16 provided at the inspection device 8, or that a pseudo scanner code and a pseudo frequency code produced by a pseudo-signal production circuit 18 are outputted to the main ultrasonic observation unit 5.

The inspection circuit 8 further includes a position sensor signal inspecting section 19 for inspecting a position sensor signal outputted from a position sensor (not shown) of the ultrasonic endoscope 3, which detects positional information at the time of performing forward and rearward driving of the ultrasonic transmitter/receiver element. The results of the inspection are outputted to the LED display panel 9.

As shown in Fig. 3, the LED display panel 9 includes: a power source LED display section 21 associated with the power source inspecting section 11, for displaying the results of the inspection on the side of the main ultrasonic observation unit 5; a transmission signal LED display section 22 associated with the transmission signal inspecting section 13; an encoder LED display section 23 associated with the encoder inspecting section 14, for display the results of the inspection on the side of the ultrasonic endoscope 3; a scanner code LED display section 24 and a frequency code LED display section 25 both associated with the code inspecting section 15; and a position sensor signal LED display section 26 associated with the position sensor signal inspecting section 19.

In the present embodiment configured as described above, when a failure has been caused in the ultrasonic endoscope 3 or the main ultrasonic observation unit 5 installed in a user facility, such as a hospital, the inspection device 8 is connected, at the user facility, with the main ultrasonic observation unit 5 or the ultrasonic endoscope 3 to carry out the following inspections (1) to (5).

Specifically, the following inspections, for example, are automatically performed:
(1) Inspection by the power source inspecting section 11 of a power source that supplies power from the main ultrasonic observation unit 5 to the ultrasonic endoscope 3, and indication of the results of the inspection on the power source LED display section 21.
(2) Inspection by the transmission signal inspecting section 13 of the transmission signal Tx and the 3D drive clock (3D CLK) transmitted from the main ultrasonic observation unit 5 to the ultrasonic endoscope 3, and indication of the results of the inspection on the transmission signal LED display section 22.
(3) Inspection by the encoder inspecting section 14 of the Phase A and the Phase Z, and indication of the results of the inspection on the encoder LED display section 22.
(4) Inspection by the code inspecting section 15 of the scanner code and the frequency code outputted from the ultrasonic endoscope 3 to the main ultrasonic observation unit 5, and indication of the results of the inspection on the scanner code LED display section 23 and the frequency code LED display section 24, respectively.
(5) Inspection by the position sensor signal inspecting section 19 of the position sensor signal outputted from the ultrasonic endoscope 3 to the main ultrasonic observation unit 5, and indication of the results of the inspection on the position sensor signal LED display section 25.

Implementing such inspections enables signal-level inspection as to which of the main ultrasonic observation unit 5 or the ultrasonic endoscope 3 is in failure and as to the conditions of the failure, and enables indication of the results of the inspection on the LED display panel 9, by only bringing the inspection device 8 to the user facility and connecting thereto the main ultrasonic observation unit 5 and the ultrasonic endoscope 3. Therefore, the conditions of the failure can be readily grasped at the user facility.

### (Second Embodiment)

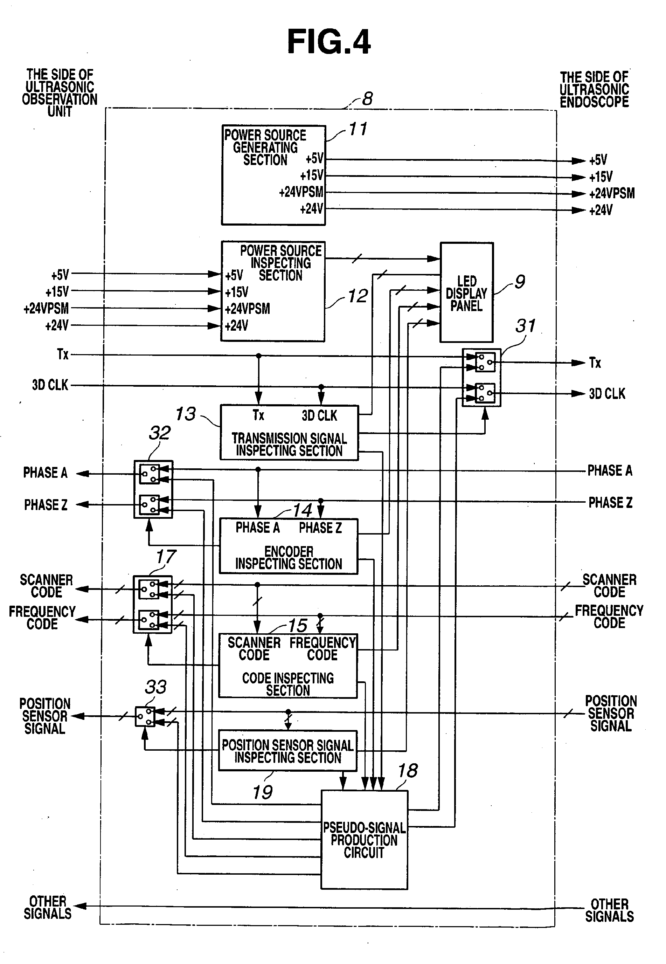

Fig. 4 is a block diagram illustrating an inspection circuit of an inspection device according to a second embodiment.

Since the second embodiment is substantially the same as the first embodiment, only the differences are explained and the same components are designated with the same references for omission of explanation.

As shown in Fig. 4, in the inspection circuit 8 of the present embodiment, it is so arranged that the pseudo-signal production circuit 18 may produce a pseudo transmission signal Tx and a pseudo 3D drive clock (3D CLK) as well as a pseudo Phase A and Phase Z and a pseudo position sensor signal, in addition to the pseudo scanner code and the pseudo frequency code, and that, for those signals having abnormality, output is switched to the pseudo-signal production circuit 18.

Specifically, the pseudo transmission signal Tx and the pseudo 3D drive clock (3D CLK) are produced based on the results of the inspection by the transmission signal inspecting section 13, while controlling a switching section 31. When there is abnormality in the transmission signal Tx and the 3D drive clock (3D CLK) from the main ultrasonic observation unit 5, the pseudo transmission signal Tx and the pseudo 3D drive clock (3D CLK) are outputted to the ultrasonic endoscope 3.

Similarly, the pseudo Phase A and Phase Z are produced based on the results of the inspection by the encoder inspecting section 14, while controlling a switching section 32. When there is abnormality in the Phase A and Phase Z from the ultrasonic endoscope 3, the pseudo Phase A and Phase Z are outputted to the main ultrasonic observation unit 5.

Further, the pseudo scanner code and the pseudo frequency code are produced based on the results of the inspection by the code inspecting section 15, while controlling the switching section 17. When there is abnormality in the scanner code and frequency code from the ultrasonic endoscope 3, the pseudo scanner code and the pseudo frequency code are outputted to the main ultrasonic observation unit 5.

Furthermore, the pseudo position sensor signal is produced based on the results of the inspection by the position sensor signal inspecting section 19, while controlling a switching section 33. When there is abnormality in the position sensor signal, the pseudo position sensor signal is outputted to the main ultrasonic observation unit 5.

As described above, in addition to the advantages of the first embodiment, the present embodiment has an advantage of enabling continuous inspection to perform finer analysis of the failure conditions by producing and outputting the individual pseudo signals from the pseudo-signal production circuit 18 even when a failure has been caused at a part of the main ultrasonic observation unit 5 or the ultrasonic endoscope 3.

The present invention is not limited to the embodiments described above, but various modifications, alterations or the like may be made without departing from the spirit and scope of the present invention.

## Claims

1. An inspection device for an ultrasonic endoscope apparatus, the device comprising:
an observation unit connecting portion for connecting a main ultrasonic observation unit;
an ultrasonic endoscope connecting portion for connecting an ultrasonic endoscope;
observation unit inspecting means for inspecting a first signal from the main ultrasonic observation unit; and
ultrasonic endoscope inspecting means for inspecting a second signal from the ultrasonic endoscope.

2. The inspection device for an ultrasonic endoscope apparatus according to claim 1, comprising electric power supplying means for supplying electric power to the ultrasonic endoscope.

3. The inspection device for an ultrasonic endoscope apparatus according to claim 1 or 2, comprising inspection results displaying means for displaying the results of inspection performed by the observation unit inspecting means and the results of inspection performed by the ultrasonic endoscope inspecting means.

4. The inspection device for an ultrasonic endoscope apparatus according to any one of claim 1, 2 or 3, comprising:
pseudo-signal producing means for producing pseudo signals, of predetermined signals, for the first signal and the second signal; and
switching means for switching the predetermined signals to the pseudo signals to effect output.

5. The inspection device for an ultrasonic endoscope apparatus according to claim 4, wherein the pseudo-signal producing means produces the pseudo signals based on the results of the inspection performed by the observation unit inspecting means or the ultrasonic endoscope inspecting means, while the switching means switches the predetermined signals to the pseudo signals to effect output.
